(19) Europäisches Patentamt
European Patent Office
Office européen des brevets

(11) Veröffentlichungsnummer : **0 075 238**
**B1**

(12) **EUROPÄISCHE PATENTSCHRIFT**

(45) Veröffentlichungstag der Patentschrift : **28.11.84**

(51) Int. Cl.³ : **C 07 C 69/16**, C 07 C 67/26

(21) Anmeldenummer : **82108424.1**

(22) Anmeldetag : **13.09.82**

# (54) Verfahren zur Herstellung von 3-Alkyl-3-acyloxy-4-hydroxy-1-butenen.

(30) Priorität : **23.09.81 DE 3137804**

(43) Veröffentlichungstag der Anmeldung : **30.03.83 Patentblatt 83/13**

(45) Bekanntmachung des Hinweises auf die Patenterteilung : **28.11.84 Patentblatt 84/48**

(84) Benannte Vertragsstaaten : **CH DE FR GB IT LI**

(56) Entgegenhaltungen :
**Keine Entgegenhaltungen.**
**KEINE**

(73) Patentinhaber : **BASF Aktiengesellschaft**
**Carl-Bosch-Strasse 38**
**D-6700 Ludwigshafen (DE)**

(72) Erfinder : **Weitz, Hans-Martin, Dr.**
**Auf dem Koeppel 40**
**D-6702 Bad Duerkheim (DE)**
Erfinder : **Fischer, Rolf, Dr.**
**Bergstrasse 98**
**D-6900 Heidelberg (DE)**

**EP 0 075 238 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 3-Alkyl-3-acyloxy-4-hydroxy-1-butenen durch Umsetzung von 3-Alkyl-3, 4-epoxy-1-butenen mit Carbonsäuren.

Es ist bekannt, daß sich z. B. 3-Methyl-3,4-epoxy-1-buten (Isoprenepoxid) in Gegenwart von Verbindungen der Formel R—OH (R = Wasserstoff, Alkyl, Acyl) und/oder Mineralsäuren zu verschiedenartigen Produkten umsetzen läßt. So entsteht beim Erhitzen mit Wasser ohne Zusatz von Säuren 2-Methylcrotonaldehyd (Tiglinaldehyd) (J. Gen. Chem USSR *13*, 481 (1943) ; C.A. *38*, 3248). Demgegenüber wird an anderer Stelle (J. Org. Chem. *35*, 1839 (1970) darauf hingewiesen, daß 3-Methyl-3,4-epoxy-1-buten mit Wasser, sogar schon bei Raumtemperatur, in 3-Methyl-3,4-dihydroxy-1-buten übergeht.

Weiterhin ist bekannt, daß 3-Methyl-3,4-epoxy-1-buten in Wasser durch Mineralsäuren, insbesondere Schwefelsäure, unter Wärmeentwicklung zu Tiglinaldehyd umgelagert wird (Ber. 66B, 335 (1933)). Auch Rhodium(I)-Komplexe, die als schwache Lewis-Säuren fungieren, lagern 3-Methyl-3,4-epoxy-1-buten zu Tiglinaldehyd um (J. Chem. Soc., Chem. Commun. *1972*, S. 491). 3-Methyl-3,4-epoxy-1-buten wird schon bei 125 °C in der Gasphase in Gegenwart von Aluminiumoxid oder Siliziumdioxid (DE-AS 26 20 927) zu Tiglinaldehyd isomerisiert. In Gegenwart von in organischen Lösungsmitteln gelösten Übergangsmetallverbindungen und Bromwasserstoff- oder Iodwasserstoffsäure entsteht dagegen 3-Methyl-2,5-dihydrofuran (US-Patent 3 932 468).

Aus Zhur Obshchei Khim. *27*, S. 2363 (C.A. *52*, 7.145) ist bekannt, daß sich 3-Methyl-3,4-epoxy-1-buten mit Methanol in Gegenwart von $BF_3$-Etherat zu 3-Methyl-3-methoxy-4-hydroxy-1-buten umsetzt.

Behandelt man 3-Methyl-3,4-epoxy-1-buten in organischen Lösungsmittel und in Gegenwart von Alkalimetalliodiden mit niederen Carbonsäuren, wie Essigsäure, so erhält man 1-Acetoxy-3-methyl-4-hydroxy-2-buten (Japanische Offenlegungsschrift 84.514/75).

Es bestand die Aufgabe, 3-Alkyl-3-acyloxy-4-hydroxy-1-butene ausgehend von leicht zugänglichen Ausgangsverbindungen mit guter Ausbeute herzustellen.

Es wurde nun überraschenderweise gefunden, daß man 3-Alkyl-3-acyloxy-4-hydroxy-1-butene der Formel

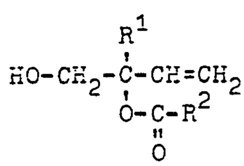

- (I)

in der $R^1$ einen Kohlenwasserstoffrest mit 1 bis 5 Kohlenstoffatomen und $R^2$ ein Wasserstoffatom oder einen Kohlenwasserstoffrest mit 1 bis 15 Kohlenstoffatomen bedeuten, dadurch herstellen kann, daß man 3-Alkyl-3,4-epoxy-1-butene der Formel

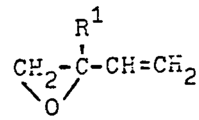

(II)

in der $R^1$ die obengenannte Bedeutung besitzt, mit Carbonsäuren der Formel $R^2$—COOH, in der $R^2$ die obengenannte Bedeutung hat, und mit Kupfersalzen von Carbonsäuren behandelt.

Als Kohlenwasserstoffreste $R^1$ kommen z. B. Alkylreste, wie Methyl-, Ethyl-, Propyl-, Butyl- und Pentylreste oder Alkenylreste, wie Propenyl- und Butenylreste in Betracht. Die Kohlenwasserstoffreste $R^2$ sind z. B. Alkyl- oder Alkylenreste mit 1 bis 15 C-Atomen oder Benzyl- oder Phenylreste.

Die Reaktion kann für den Fall der Umsetzung von 3-Methyl-3,4-epoxy-1-buten mit Essigsäure durch die folgenden Formeln wiedergegeben werden :

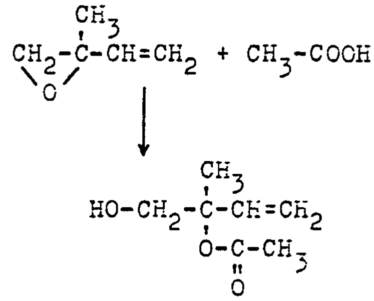

Nach dem Stand der Technik war nicht vorauszusehen, ob man ausgehend von 3-Methyl-3,4-epoxy-1-buten bei der erfindungsgemäßen Umsetzung mit einer mittelstarken Säure wie Essigsäure als Hauptprodukt Tiglinaldehyd, 3-Methyl-2,5-dihydrofuran, 3-Methyl-1-acetoxy-4-hydroxy-2-buten oder das gewünschte 3-Methyl-3-acetoxy-4-hydroxy-1-buten erhalten würde.

Als Ausgangsstoffe der Formel II sind z. B. 3-Methyl-, 3-Ethyl-, 3-n-Propyl-, 3-i-Propyl-, 3-n-Butyl-, 3-tert.-Butyl-, 3-n-Pentyl-, 3-Propenyl-, 3-Butenyl-3,4-epoxy-1-buten geeignet.

Die genannten Ausgangsverbindungen II lassen sich beispielsweise durch Epoxidierung der entsprechenden 1,3-Diene mit Persäuren (DE-OS 27 34 242), mit Hydroperoxiden (J. Org. Chem. *35*, 1839 (1970) oder mit Wasserstoffperoxid (DE-AS 20 12 049) sowie nach der Halogenhydrinmethode (J. Org. Chem. *25*, 1673 (1960) herstellen.

Als Carbonsäuren sind z. B. Ameisensäure, Essigsäure, Propionsäure, Buttersäure, Valeriansäure, Caprinsäure, Laurinsäure, Ölsäure, Palmitinsäure, Cyclohexancarbonsäure, Benzoesäure oder Phenylessigsäure geeignet. Als Kupfersalze verwendet man die Kupfersalze der genannten Carbonsäuren.

Eine bevorzugte Ausführungsform des Verfahrens besteht darin, daß man das Epoxid zu der flüssigen Carbonsäure hinzufügt, bei Temperaturen zwischen dem Erstarrungspunkt der jeweiligen Carbonsäure und 90 °C und in Gegenwart der Kupfersalze von Carbonsäuren arbeitet. Hierdurch wird die Bildung von Tiglinaldehyd zurückgedrängt (siehe Versuchsergebnisse in der Tabelle). Der Erstarrungspunkt der Carbonsäure kann dadurch erniedrigt werden, daß man Gemische aus der jeweiligen Carbonsäure und einem unter den Reaktionsbedingungen inerten Lösungsmittel verwendet.

Die Umsetzung kann beispielsweise so durchgeführt werden, daß man die jeweilige Carbonsäure, die im Überschuß gleichzeitig als Lösungsmittel dienen kann, auf die jeweilige Reaktionstemperatur bringt und dann das Epoxid und das Kupfersalz hinzufügt. Nach der notwendigen Reaktionszeit wird die nicht umgesetzte Carbonsäure abdestilliert, der Rückstand wird zur Gewinnung des 3-Alkyl-3-acyloxy-4-hydroxy-1-butens fraktioniert destilliert.

Die Umsetzung wird z. B. bei Temperaturen von −20 bis 150 °C, insbesondere 0 bis 90 °C, drucklos oder unter Druck, 0,5 bis 20 Stunden lang durchgeführt. Das Molverhältnis zwischen dem Epoxid und der Carbonsäure beträgt zweckmäßig 1 : 1 bis 1 : 30, insbesondere 1 : 1,1 bis 1 : 20. Pro Mol Epoxid verwendet man z. B. 0,01 bis 1, insbesondere 0,05 bis 0,2 Mol der genannten Kupfersalze.

Als unter den Reaktionsbedingungen inerte Lösungsmittel, die insbesondere im Falle von hochschmelzenden Carbonsäuren verwendet werden, sind z. B. Carbonsäureester, wie Essigsäuremethylester, chlorierte Kohlenwasserstoffe, wie Methylenchlorid, Chloroform, Tetrachlorkohlenstoff, 1,2-Dichlorethan, Kohlenwasserstoffe, wie Alkane, Alkene, Alkine, Benzol- und alkylierte Benzole, Ether, wie Diethylether, Tetrahydrofuran, Dioxan verwendbar. Pro Mol Ausgangsverbindung verwendet man 0,01 bis 30 Mol, insbesondere 0,1 bis 20 Mol des jeweiligen Lösungsmittels.

Die nach dem Verfahren der Erfindung erhältlichen 3-Alkyl-3-acyloxy-4-hydroxy-1-butene sind wertvolle Ausgangsstoffe für die Herstellung von Pflanzenschutzmitteln, pharmazeutischen Produkten der Vitamin-Vorprodukte. So läßt sich z. B. 3-Methyl-3-acetoxy-4-hydroxy-1-buten zu 2-Methyl-2-acetoxy-3-butenal oxidieren, das sich nach den Angaben der DE-AS 12 97 597 und der DE-OS 28 40 125 zu dem Vitamin-A-Vorprodukt 2-Methyl-4-acetoxy-2-butenal umlagern läßt. Verseifung der Acetoxygruppe und Oxidation der primären OH-Gruppe führt vom gleichen Ausgangsprodukt zu Vinylmilchsäure, einem Vorprodukt für ein wichtiges Fungizid (DE-OS 22 07 576).

## Beispiele 1 bis 15

(Beispiele 1, 3, 5, 6, 7, 8, 10, 12, 14 und 15 sind Vergleichsbeispiele)

Das verwendete 3-Methyl-3,4-epoxy-1-buten wurde nach den Angaben in Journal of Organic Chemistry, Band 25, Seite 1673 ff. hergestellt. Zu 100 g Eisessig, in denen gegebenenfalls Zusätze (siehe Tabelle) gelöst sind, werden bei der angegebenen Temperatur unter Rühren innerhalb von 10 Minuten 8,4 g 3-Methyl-3,4-epoxy-1-buten zugetropft. Man rührt 30 Minuten bei dieser Temperatur nach und analysiert dann das Reaktionsprodukt gaschromatographisch (4 m Carbowax 20 M-Säule). Die Zusammensetzung des Reaktionsgemisches ist in der Tabelle angegeben.

## Beispiel 16

Zu 150 g Eisessig, in denen 12 g $Cu(CH_3COO)_2 \cdot H_2O$ gelöst sind, werden bei 25 ± 2 °C unter Rühren innerhalb von 30 Minuten 50,4 g 3-Methyl-3,4-epoxy-1-buten zugetropft. Man rührt 2 Stunden bei dieser Temperatur nach und zieht dann bei Wasserstrahlvakuum am Rotationsverdampfer die überschüssige Essigsäure ab. Durch fraktionierte Destillation des Rückstandes werden 59,7 g 3-Methyl-3-acetoxy-4-hydroxy-1-buten vom Siedepunkt 74 °C/13 mbar, $n_D^{20}$=1,441 8, erhalten (69 %, bezogen auf eingesetztes Epoxid).

(Siehe Tabelle Seite 4 f.)

Tabelle

Umsetzung von 3-Methyl-3,4-epoxy-1-buten mit Essigsäure sowie mit und ohne Kupfersalzen

| Versuchs-Nr. | Temperatur [°C] | Zusatz [g] | I | II | III | IV | Tiglinaldehyd |
|---|---|---|---|---|---|---|---|
| | | | Mol-%, bezogen auf eingesetztes 3-Methyl-3,4-epoxy-1-buten | | | | |
| 1[1)] | 10 | - | 52 | 0,1 | 15 | 0,2 | 13 |
| 2[1)] | | 2 g $Cu(OAc)_2 \cdot H_2O$ | 75 | 0,3 | 8 | 0,1 | 6 |
| 3 | 25 | - | 52 | 0,2 | 16 | 0,2 | 15 |
| 4 | | 2 g $Cu(OAc)_2 \cdot H_2O$ | 74 | 2 | 8 | 0,1 | 5 |
| 5 | | 2,4 g $Cu(NO_3)_2 \cdot 3\,H_2O$ | 56 | 1 | 8 | 0,5 | 11 |
| 6 | | 1,9 g CuI | 58 | 1 | 17 | 0,2 | 14 |
| 7 | | 0,98g KOAc | 53 | 0,4 | 18 | 0,1 | 16 |
| 8 | 40 | - | 51 | 0,2 | 16 | 0,2 | 17 |
| 9 | | 2 g $Cu(OAc)_2 \cdot H_2O$ | 58 | 9 | 12 | 0,2 | 6 |
| 10 | 60 | - | 50 | 0,1 | 17 | 0,2 | 20 |
| 11 | | 2 g $Cu(OAc)_2 \cdot H_2O$ | 57 | 17 | 10 | 0,4 | 4 |
| 12 | 80 | - | 49 | 0,1 | 17 | 1 | 23 |
| 13 | | 2 g $Cu(OAc)_2 \cdot H_2O$ | 57 | 19 | 8 | 1 | 4 |
| 14 | 100 | - | 44 | 0,5 | 15 | 3 | 27 |
| 15 | 118 | - | 37 | 2 | 12 | 9 | 32 |

1) In den 100 g Essigsäure sind 10 Gew.% Essigsäureethylester enthalten.
I = 3-Methyl-3-acetoxy-4-hydroxy-1-buten
II = 3-Methyl-3,4-diacetoxy-1-buten
III = cis + trans 2-Methyl-1-hydroxy-4-acetoxy-2-buten
IV = cis + trans 2-Methyl-1,4-diacetoxy-2-buten
V = 2-Methyl-crotonaldehyd (Tiglinaldehyd)

**Ansprüche**

1. Verfahren zur Herstellung von 3-Alkyl-3-acyloxy-4-hydroxy-1-butenen der Formel

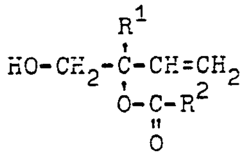
(I)

in der $R^1$ einen Kohlenwasserstoffrest mit 1 bis 5 Kohlenstoffatomen und $R^2$ ein Wasserstoffatom oder einen Kohlenwasserstoffrest mit 1 bis 15 Kohlenstoffatomen bedeuten, dadurch gekennzeichnet, daß man 3-Alkyl-3,4-epoxy-1-butene der Formel

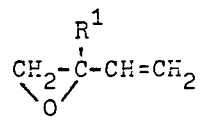
(II)

in der $R^1$ die obengenannte Bedeutung besitzt, mit Carbonsäuren der Formel $R^2$—COOH, in der $R^2$ die obengenannte Bedeutung hat, und mit Kupfersalzen von Carbonsäuren behandelt.

2. Verfahren nach Anspruch 1, dadurch gekennzeichnet, daß man die Umsetzung bei Temperaturen zwischen − 20 und + 90 °C durchführt.

**Claims**

1. A process for the preparation of a 3-alkyl-3-acyloxy-4-hydroxybut-1-ene of the formula

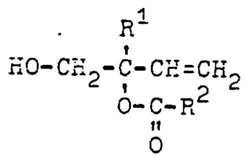
(I)

where $R^1$ is a hydrocarbon radical of 1 to 5 carbon atoms and $R^2$ is hydrogen or a hydrocarbon radical of 1 to 15 carbon atoms, wherein a 3-alkyl-3,4-epoxybut-1-ene of the formula

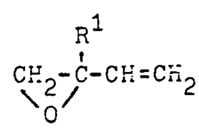
(II)

where $R^1$ has the above meaning, is treated with a carboxylic acid of the formula $R^2$—COOH, where $R^2$ has the above meanings, and with a copper salt of a carboxylic acid.

2. A process as claimed in Claim 1, wherein the reaction is carried out from − 20 to + 90 °C.

**Revendications**

1. Procédé de préparation de 3-alkyl-3-acyloxy-4-hydroxy-1-butènes de formule

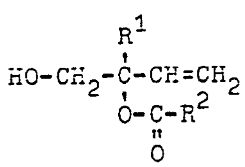
(I)

dans laquelle $R^1$ représente un reste hydrocarboné en C1-C5 et $R^2$ un atome d'hydrogène ou un reste hydrocarboné en C1-C15, caractérisé en ce que l'on traite des 3-alkyl-3,4-époxy-1-butènes de formule

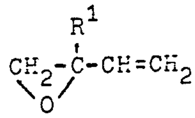

(II)

dans laquelle $R^1$ a la signification indiquée ci-dessus, par des acides carboxyliques de formule $R^2$—COOH dans laquelle $R^2$ a la signification indiquée ci-dessus, et des sels de cuivre des acides carboxyliques.

2. Procédé selon la revendication 1, caractérisé en ce qu'on effectue la réaction à des températures de − 20 à + 90 °C.